(19) Europäisches Patentamt
European Patent Office
Office européen des brevets

(11) **EP 4 574 205 A1**

(12) **EUROPÄISCHE PATENTANMELDUNG**

(43) Veröffentlichungstag:
    25.06.2025  Patentblatt 2025/26

(21) Anmeldenummer: 23219623.8

(22) Anmeldetag: 22.12.2023

(51) Internationale Patentklassifikation (IPC):
    **A61N 5/10** (2006.01)  **G01R 33/48** (2006.01)

(52) Gemeinsame Patentklassifikation (CPC):
    **A61N 5/1064; A61N 5/1048; A61N 5/1071;**
    **G01R 33/4804; G01R 33/4808**

(84) Benannte Vertragsstaaten:
    **AL AT BE BG CH CY CZ DE DK EE ES FI FR GB**
    **GR HR HU IE IS IT LI LT LU LV MC ME MK MT NL**
    **NO PL PT RO RS SE SI SK SM TR**
    Benannte Erstreckungsstaaten:
    **BA**
    Benannte Validierungsstaaten:
    **KH MA MD TN**

(71) Anmelder: **Siemens Healthineers AG**
    **91301 Forchheim (DE)**

(72) Erfinder: **Popescu, Stefan**
    **91056 Erlangen (DE)**

(74) Vertreter: **Siemens Healthineers**
    **Patent Attorneys**
    **Postfach 22 16 34**
    **80506 München (DE)**

(54) **VERFAHREN UND VORRICHTUNG ZUR ECHTZEIT-ÜBERWACHUNG EINER APPLIZIERTEN STRAHLENDOSIS**

(57)    Die Erfindung betrifft ein Verfahren zur Echtzeitüberwachung einer von einer Bestrahlungsvorrichtung applizierten Strahlendosis in einem Zielgebiet mittels eines Magnetresonanztomographen, sowie ein System aus dem Magnetresonanztomographen und der Bestrahlungsvorrichtung. Die Bestrahlungsvorrichtung ist ausgebildet, die Strahlung in einer in der Intensität im akustischen Frequenzbereich modulierten Form abzugeben. Die Bestrahlungsvorrichtung strahlt eine modulierte Strahlung in das Zielgebiet und der Magnetresonanztomograph erfasst währenddessen eine Magnetresonanzabbildung des Zielgebiets mit einer Sequenz, die sensitiv auf eine Amplitude einer Auslenkung eines Gewebes in dem Zielgebiet.

FIG 1

EP 4 574 205 A1

**Beschreibung**

Unabhängig vom grammatikalischen Geschlecht eines bestimmten Begriffes sind Personen mit männlicher, weiblicher oder anderer Geschlechteridentität mit umfasst.

Die Erfindung betrifft ein Verfahren zur Echtzeitüberwachung einer von einer Bestrahlungsvorrichtung applizierten Strahlendosis in einem Zielgebiet mittels eines Magnetresonanztomographen sowie ein System aus einem Magnetresonanztomographen und einer Bestrahlungsvorrichtung. Die Bestrahlungsvorrichtung ausgebildet ist, die Strahlung gepulst abzugeben. Der Magnetresonanztomograph ist ausgebildet, eine Magnetresonanzabbildung des Zielgebiets während der Bestrahlung zu erfassen.

Bei einer Strahlentherapie wird versucht, ein Ziel, beispielsweise einen Tumor, mit einer definierten Mindestdosis zu bestrahlen und gleichzeitig umgebendes Gewebe möglichst wenig zu schädigen. Dazu werden in Vorbereitung der Bestrahlung die genaue Lage der Organe erfasst, beispielsweise mit einer Computertomographie oder einer Magnetresonanztomographie. Anhand dieser Daten werden mit Modellrechnungen ein Bestrahlungsplan erstellt, in dem die Bestrahlungsrichtungen und Intensitäten für die Behandlung ermittelt werden. Aufgrund von Bewegungen einzelner Organe und auch Fehler in den Annahmen, z.B. über individuelle Eigenschaften der einzelnen Gewebearten, weichen die tatsächlich applizierten Strahlendosen von diesen Modellrechnungen ab.

Aus dem Artikel "Real-time, volumetric imaging of radiation dose delivery deep into the liver during cancer treatment | Nature Biotechnology"(https://www.nature.com/articles/s41587-022-01593-8) ist ein Verfahren bekannt, mittels der durch die Strahlung bzw. deren Impuls- und Energieübertrag erzeugten Schallwellen im Gewebe eine Dosisverteilung zu erfassen.

Die Druckschrift DE 10 2012 211581 A1 beschreibt ein Verfahren zur Elastographie. Das Verfahren umfasst das Einstrahlen eines Hochfrequenz-Pulses zum Manipulieren einer Transversalmagnetsierung in dem bestimmten Bereich (30) und das Einstrahlen mindestens eines weiteren Hochfrequenz-Pulses mit einer Ortsselektivität der Amplitude zum Erzeugen von Scherwellen in dem bestimmten Bereich. Das Verfahren umfasst das Detektieren eines Magnetresonanz-Signals aus dem bestimmten Bereich und das Bestimmen einer die Gewebeelastizität beschreibenden Größe in dem bestimmenden Bereich, basierend auf dem Magnetresonanz-Signal.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt.

Es ist eine Aufgabe der vorliegenden Erfindung, eine Strahlentherapie zuverlässiger zu gestalten.

Die Aufgabe wird durch ein erfindungsgemäßes Verfahren nach Anspruch 1 und ein erfindungsgemäßes System nach Anspruch 7 gelöst.

Das erfindungsgemäße Verfahren ist zur Echtzeitüberwachung einer von einer Bestrahlungsvorrichtung applizierten Strahlendosis in einem Zielgebiet mittels eines Magnetresonanztomographen vorgesehen. Als Echtzeitüberwachung wird dabei eine Überwachung angesehen, die noch während einer einzelnen Bestrahlungs-Sitzung die bis dahin applizierte Dosis erfasst und es ermöglicht, diese zu verändern bzw. aufgrund der erfassten Dosis die weitere Bestrahlung zu verändern. Vorzugsweise wird die Dosis für Zeitabschnitte kleiner als 10 s, 1 s oder 0,1 s erfasst. Beispielsweise wird die Dosis für jeweils einzelne der nachfolgend erläuterten Strahlungspulse ermittelt.

Die Bestrahlungsvorrichtung ist ausgebildet, ionisierende Strahlung zur Therapie z.B. von Tumoren abzugeben. Unter ionisierende Strahlung sind zum einen Photonen mit einer Energie größer als 1 keV, 10 keV, 100 keV oder 1 MeV zu verstehen. Derartige Photonen können beispielsweise von einer Röntgenröhre oder einem Beschleuniger für geladene Teilchen erzeugt werden, aber auch von einem radioaktiven Element. Denkbar sind aber auch ionisierende Partikelstrahlen mit hochenergetischen Beta-Teilchen bzw. Elektronen, Protonen, Alpha-Teilchen oder auch schwerere Ionen denkbar. Unter hochenergetisch werden dabei Partikel mit kinetischer Energie größer als 1 keV, 10 keV, 100 keV oder 1 MeV angesehen.

Die Bestrahlungsvorrichtung ist dabei ausgebildet, die abgegebene Strahlung in einer in der Intensität modulierten Form abzugeben. Darunter ist zu verstehen, dass sich die Strahlungsleistung der Quelle mit der Zeit verändert, vorzugsweise um mindestens 10 %, 30 % oder 50%. Es ist auch denkbar, dass sich die Intensität um bis zu 100% verändert, mit anderen Worten, die Strahlung gepulst abgegeben wird. Die Frequenz der Änderungen ist dabei im akustischen Frequenzbereich, d.h. vorzugsweise im Bereich von 1 Hz bis 100 kHz oder 10 Hz bis 10 kHz. Insbesondere ist die Frequenz so gewählt, dass ein Impuls- und/oder Energieübertrag der Strahlung eine mechanische Bewegung bzw. Auslenkung im Gewebe im Zielgebiet hervorrufen kann, die dann auch mit einer Magnetresonanzbildgebung erfasst werden kann. Denkbar sind dabei Scher- bzw. Transversalschwingung als auch Kompessions- bzw. Longitudinalwellen.

Die Auslenkung kann eine für einen Strahlenpuls einmalige Auslenkung von Volumenelementen des Gewebes mit anschließender Rückkehr in die Ruhelage angesehen, in Form einer exponentiell gedämpften Schwingung oder auch auf asymptotische Art. Denkbar ist aber auch eine periodische Bewegung in Form einer Schwingung.

Die Frequenz der Modulation bzw. die Strahlenpulse können auch insbesondere mit einer Frequenz einer Bilderfassungssequenz des Magnetresonanztomographen übereinstimmen bzw. mit deren Ablauf synchronisiert sein, die, wie nachfolgend erläutert, beim Erfassen einer Magnetresonanzabbildung genutzt wird. Beispielsweise kann die Bestrahlung gepulst sein und der Puls in einem vorbestimmten zeitlichen Abstand zu einem Referenzpunkt in der Sequenz, beispielsweise zu einem RF-Puls und/oder einem Gradientenpuls. Denkbar ist aber auch, dass der vorbestimmte zeitliche Abstand in einer vorbestimmten Weise variiert, beispielsweise um eine Phasenlage bei der Abtastung zu verändern. Die Synchronisation kann dabei vorzugsweise über eine Signalverbindung zwischen Magnetresonanztomograph und Bestrahlungsvorrichtung, gesteuert von dem Magnetresonanztomographen oder der Bestrahlungsvorrichtung, oder in einem gegenseitigen Synchronisierungsprotokoll. Dabei können besondere Sicherheitsanforderungen der Bestrahlungsvorrichtung berücksichtigt sein, um eine Gefährdung des Patienten durch eine zu lange Bestrahlung auszuschließen.

Der Magnetresonanztomograph bzw. das System aus Magnetresonanztomograph und Bestrahlungsvorrichtung ist weiterhin ausgebildet, eine Magnetresonanzabbildung des Zielgebiets während der Bestrahlung zu erfassen. Mit anderen Worten, der Magnetresonanztomograph und die Bestrahlungsvorrichtung sind so relativ zueinander angeordnet, dass die Bestrahlung eines Patienten erfolgen kann, während sich der Patient in einem Bilderfassungsbereich des Magnetresonanztomographen, z.B. im Patiententunnel, befindet. Der Magnetresonanztomograph muss darüber hinaus einen Zugang für die Strahlung zu dem Bilderfassungsbereich aufweisen.

Das Verfahren weist den Schritt auf, eine modulierte Strahlung mit der Bestrahlungsvorrichtung in das Zielgebiet einzustrahlen.

Gleichzeitig wird in einem Schritt des Verfahrens eine Magnetresonanzabbildung des Zielgebiets mit dem Magnetresonanztomographen erfasst. Unter gleichzeitig ist dabei zu verstehen, dass zumindest Teile der Bilderfassungssequenz erfolgen, während die Bestrahlung erfolgt. Die Sequenz ist dabei so ausgebildet, dass sie eine Amplitude einer Auslenkung bzw. Schwingung eines Gewebes im Zielgebiet mit einer Frequenz der Modulation der Bestrahlungsmodulation erfassen kann, also das Magnetresonanzsignal abhängig von der Amplitude der Auslenkung ist. Beispielhafte Sequenzen zum Erfassen der Auslenkung sind unter anderem GRE-EPI-, Spin-Echo- oder eine modifizierte EPI-Sequenz, wie sie nachfolgend zu den Figuren erläutert sind. Der Begriff "gleichzeitig" ist vorzugsweise so zu verstehen, dass die Bestrahlung bzw. die Pulse mit der Bilderfassungssequenz in vorbestimmter Weise verschachtelt ist. Vorzugsweise erfolgt eine Synchronisation von Einstrahlen und Bilderfassung. Dies kann beispielsweise über eine Signalverbindung zwischen Bestrahlungsvorrichtung und Magnetresonanztomograph erfolgen. Denkbar ist, dass eine der Vorrichtungen der Master ist, aus Sicherheitsgründen vorzugsweise die Bestrahlungsvorrichtung, oder die Synchronisierung zwischen beiden erfolgt über ein Synchronisationsprotokoll im Austausch oder durch eine weitere Synchronisationseinheit.

In einem weiteren Schritt ermittelt eine Steuerung, Teil des Magnetresonanztomographen oder auch separat, eine Dosisverteilung der Strahlung in dem Zielgebiet aus der erfassten Magnetresonanzabbildung bzw. in Abhängigkeit von der in der Magnetresonanzabbildung erfassten Auslenkung bzw. deren Amplitude. Die Dosisverteilung kann beispielsweise in Form einer 2- oder 3-dimensionalen Karte als eine räumliche Verteilung bzw. ortsaufgelöste Information über die Strahlendosis angegeben werden.

Die applizierte Strahlendosis steht in einem funktionalen Zusammenhang mit der Amplitude der erfassten Auslenkung, indem die Auslenkung durch die Wirkung der Strahlung erfolgt. Denkbar ist eine Auslenkung infolge einer thermischen Wirkung, also eine Ausdehnung durch Erwärmung. Die Ausdehnung bewirkt eine Verschiebung von Gewebe aus einem Mittelpunkt der Erwärmung heraus. Denkbar ist auch ein direkter Impulsübertrag von der Strahlung auf das Gewebe induziert wird, da zum einen auch hochenergetische Photonen einen Impuls aufweisen, insbesondere aber Partikelstrahlen aus Teilchen mit Ruhemasse. Die Auslenkung kann beispielsweise auch auf mathematisch komplexe Weise von der Strahlendosis abhängen, insbesondere bei der thermischen Wirkung. Mittels einer Kalibrierung oder einer mathematischen Modellierung kann daraus eine Strahlungsdosis bestimmt werden. Da die Auslenkung unter anderem vom der Leistung und/oder dem Impuls des Strahls abhängt, ergibt sich die Dosis durch ein Intergral der Auslenkung über die Zeit. Diese Integration kann mathematisch, oder vorzugsweise bereits durch die zum Erfassen genutzte Sequenz ermittelt werden, wie nachfolgend zu den Figuren näher erläutert wird. So kann aus der erfassten Amplitudenverteilung eine räumliche Verteilung bzw. Karte der applizierten Strahlendosis ermittelt werden, beispielsweise durch die Steuerung des Magnetresonanztomographen oder eine andere Steuerung.

Auf vorteilhafte Weise nützt das erfindungsgemäße Verfahren die strahlungsinduzierte Auslenkung und deren Erfassung mittels einer Magnetresonanzsequenz, um eine in einem Patienten applizierte Strahlendosis in Echtzeit und ortsbezogen zu ermitteln.

Weitere vorteilhafte Ausführungsformen sind zu den Unteransprüchen angegeben.

In einer bevorzugten Ausführungsform des erfindungsgemäßen Verfahrens weist das Verfahren weiterhin den Schritt auf, eine Referenz-Magnetresonanzabbildung des Zielgebiets ohne gleichzeitiges Einstrahlen durch die Bestrahlungsvorrichtung mittels des Magnetresonanztomographen zu erfassen. Vorzugsweise erfolgt das Erfassen der Referenz- Magnetresonanzabbildung dabei mit einer bis auf das Einstrahlen identischen Sequenz.

Die Referenz-Magnetresonanzabbildung wird anschließend in dem Schritt des Ermittelns der Dosisleistung genutzt. Der Effekt der strahleninduzierten Auslenkung und deren Auswirkungen sind selbst bei optimierten Sequenzen gering und können durch andere Effekte wie B0- oder B1-Inhomogentitäten überlagert sein. Auf vorteilhafte Weise können durch eine Differenzbildung von Bilddaten mit und ohne Einstrahlen diese störenden Effekte auf vorteilhafte Weise reduziert werden.

In einer denkbaren Ausführungsform des erfindungsgemäßen Verfahrens wird bei dem Schritt des Erfassens der Magnetresonanzabbildung und dem Schritt des Erfassens der Referenz-Magnetresonanzabbildung eine Sequenz mit Motion-Encoding-Gradienten (MEG) verwendet. Ein Motion-Encoding-Gradient ist ausgelegt, Kernspins einer Schicht bzw. eines Volumenelements in Phase und/oder Amplitude deren Präzession zu verändern, sodass mittels Messung der Magnetresonanzsignale eine räumliche Verschiebung des Volumenelements bzw. der Schicht erfasst werden kann. Vorzugsweise wird dazu ein Gradient entlang der Auslenkung genutzt, sodass Kernspins, die einer Auslenkung unterliegen, zumindest zeitweise einem anderen Magnetfeld B0 plus der Gradientenstärke mal einer Amplitude der Auslenkung ausgesetzt sind und so eine Phasenveränderung in Bezug nicht (in Richtung des Gradienten) ausgelenkten Kernspins ausgesetzt sind. Sequenzen mit derartigen Gradienten sind beispielsweise aus den anfangs genannten Druckschriften zur Elastographie bekannt. Vorzugsweise ist der Motion-Encoding-Gradient deshalb im Wesentlichen parallel zu der Richtung der Auslenkung. Denkbar ist es auch, zum Erfassen der räumlichen thermischen Ausdehnung Bilderfassungen mit Gradienten in drei Dimensionen anzuwenden. Eine Bilderfassung mit dem bzw. den angelegten MEG erfolgt, während der Strahl in das Zielgebiet einfällt.

Denkbar sind auch für die Diffusionsmessung verwendete Magnetresonanz-Sequenzen oder Spinlabeling-Sequenzen wie für Fluss-MRI.

Dabei erfolgt in dem Schritt des Erfassens einer Magnetresonanzabbildung und/oder dem Schritt des Erfassens einer Referenz-Magnetresonanzabbildung das Erfassen mit invertierter Polarität eines Motion-Encoding-Gradienten.

Auf vorteilhafte Weise sorgt ein Puls mit invertierter Polarität beim Erfassen als sogenannter bipolarer oder "balanced" Motion Encoding Gradient dafür, dass Artefakte, wie z.B. durch die Suszeptibilität von Metallen oder von Suszeptibilitätssprüngen zwischen unterschiedlichen Gewebearten oder Gewebe und Luft verursacht, sich aufgrund der umgekehrten Vorzeichen sich aufheben und lediglich die bewegungsbedingten Effekte verbleiben.

In einer möglichen Ausführungsform des erfindungsgemäßen Verfahrens weist das Verfahren weiterhin den Schritt auf, mittels einer Bilderfassung durch den Magnetresonanztomographen mit einer temperatursensitiven Sequenz eine Temperatur-Magnetresonanzabbildung des Zielgebiets zu erfassen. Dies kann mittels einer Auswertung der temperaturabhängigen Veränderung der Larmorfrequenz oder auch einer temperaturabhängigen Veränderung der T1-Relaxationeszeit ermittelt werden. In einem weiteren Schritt wird eine Temperaturkarte des Zielgebiets aus der Temperatur-Magnetresonanzabbildung ermittelt.

Auf vorteilhafte Weise kann zum einen eine mittels der Bewegung ermittelte Strahlendosis durch deren Wärmewirkung verifiziert werden. Auch ist die Wirkung der Strahlung auf den Tumor temperaturabhängig, sodass die Wirkung der Strahlendosis besser abgeschätzt werden kann und die Strahlendosis bei gleichem therapeutischem Erfolg optimiert bzw. verringert werden kann.

In einer denkbaren Ausführungsform des erfindungsgemäßen Verfahrens wird aus einer der erfassten Magnetresonanzabbildungen eine Position eines Zielgewebes, beispielsweise eines Tumors, aber auch eines zu schützenden benachbarten Organs, ermittelt wird und eine weitere Bestrahlung in Abhängigkeit von der ermittelten Position erfolgt. Durch natürliche Bewegung des Körpers, beispielsweise Atmung, Herzschlag oder Peristaltik, aber auch durch willkürliche Bewegungen, kann sich die Lage eines Tumors im Lauf einer Behandlung und insbesondere zwischen unterschiedlichen Behandlungsterminen durch ein behandlungsbedingtes Schrumpfen auch bei sorgfältiger externer Markierung und Positionierung verändern, sodass zum einen die Bestrahlung nicht oder nicht vollständig das Ziel, z.B. den Tumor, trifft und stattdessen benachbarte wichtige Organe schädigt.

Indem der Magnetresonanztomograph mit der Bilderfassung für die Dosisermittlung gleichzeitig auch eine Spinverteilung für eine "normale" Abbildung erfasst, z.B. wenn eine Auswertung ohne Differenzbildung erfolgt, kann auf vorteilhafte Weise auf diese quasi Echtzeit-Bildgebung zurückgegriffen werden und die Bestrahlung auf vorteilhafte Weise angepasst werden, sei es durch Neuausrichtung der Bestrahlungsvorrichtung, des Patienten oder auch nur durch eine Unterbrechung, z.B. bei einer Atmung oder Herzschlag, bis der Tumor und/oder benachbarte Organe wieder die vorbestimmte Position der Bestrahlungsplanung eingenommen haben.

In einer möglichen Ausführungsform des erfindungsgemäßen Verfahrens weist das Verfahren weiterhin den Schritt auf, mittels eines Hochfrequenzsignals des Magnetresonanztomographen eine vorbestimmte Erwärmung in dem Zielgebiet zu erzielen. Die zur Anregung der Kernspins notwendigen Hochfrequenzpulse führen immer zu einer Erwärmung des Körpergewebes durch die absorbierte Hochfrequenzenergie. Durch geeignetes Design der Hochfrequenzpulse, beispielsweise durch Emission über mehrere Antennen zur räumlichen Veränderung, oder zusätzliche Hochfrequenzpulse kann die Erwärmung beeinflusst werden, um in einem vorbestimmten Zielgebiet eine vorbestimmte Temperaturanhebung zu erzielen.

Auf vorteilhafte Weise kann durch eine Temperaturerhöhung die Wirkung der Bestrahlung auf das Gewebe optimiert werden. Mit der bereits dargestellten Thermometrie mittels Magnetresonanzabbildung kann darüber hinaus dieser Effekt genauer eingestellt und überwacht werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:

Fig. 1    eine schematische Darstellung einer erfindungsgemäßen Vorrichtung;

Fig. 2    einen schematischen Ablaufplan eines erfindungsgemäßen Verfahrens;

Fig. 3    eine schematische Darstellung der Strahlungswirkung auf Gewebe eines Patienten;

Fig. 4    einen zeitlichen Zusammenhang zwischen Strahlung und Auslenkung;

Fig. 5    einen beispielhaften schematischen zeitlichen Verlauf einer GRE-EPI-Sequenz zur Verwendung in der Erfindung;

Fig. 6    einen beispielhaften schematischen zeitlichen Verlauf einer modifizierten SE Sequenz zur Verwendung in der Erfindung;

Fig. 7    einen beispielhaften schematischen zeitlichen Verlauf einer modifizierten EPI-Sequenz zur Verwendung in der Erfindung.

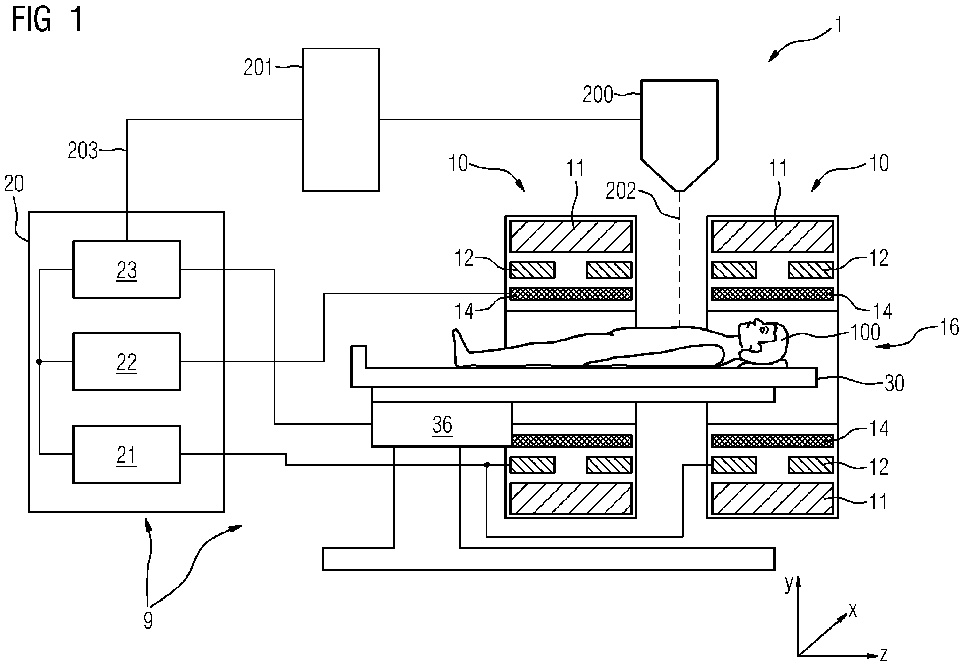

Fig. 1 zeigt eine schematische Darstellung einer beispielhaften Ausführungsform einer erfindungsgemäßen Vorrichtung zur Echtzeitüberwachung einer von einer applizierten Strahlendosis.

Die Vorrichtung der Fig. 1 weist im Wesentlichen einen Magnetresonanztomographen 9 und eine Bestrahlungsvorrichtung 200 auf, deren Steuereinheiten 20, 201 über eine Signalverbindung 203 miteinander verbinden sind, um die Echtzeitüberwachung des erfindungsgemäßen Verfahrens auszuführen.

Die Magneteinheit 10 des Magnetresonanztomographen 9 weist einen Feldmagneten 11 auf, der ein statisches Magnetfeld B0 zur Ausrichtung von Kernspins von Proben bzw. Patienten 100 in einem Aufnahmebereich erzeugt. Die Magneteinheit 10 in Fig. 1 ist dabei beispielhaft als horizontale Split-Magnet dargestellt, d.h. die Magneteinheit 10 ist in zwei Teile aufgeteilt bzw. unterteilt, sodass die Bestrahlungsvorrichtung 200 freien Zugang für die Bestrahlung zu dem Patienten 100 hat. Es sind auch andere Ausführungsformen der Magneteinheit 10 denkbar, die Zugang für die Bestrahlung und gleichzeitig eine Bilderfassung im Zielgebiet ermöglichen, beispielsweise auch vertikale Split-Magnet-Systeme oder einteilige Magneteinheiten 10 mit entsprechenden Durchbrüchen. Grundsätzlich sind auch Strahlungsquellen als Bestrahlungsvorrichtung 200 innerhalb der Magneteinheit 1 denkbar, sofern deren Strahlungsintensität erfindungsgemäß modulierbar sind und sie kompatibel mit der Bilderfassung sind.

Der Aufnahmebereich des Magnetresonanztomographen ist in einem Patiententunnel 16 angeordnet, der sich in einer Längsrichtung 2 durch die Magneteinheit 10 erstreckt. Der Patiententunnel 16 ist dabei zumindest, wie zuvor bereits erläutert, vorzugsweise zumindest teilweise unterbrochen, sodass der Patient zugänglich für eine Bestrahlung durch die Bestrahlungsvorrichtung 200 möglich ist. Der Aufnahmebereich ist dabei in einem Bereich, der auch das Zielgebiet der Bestrahlung in dem Patienten 100 umfasst.

Der Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die Felder mit zueinander orthogonalen Gradienten in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in den Aufnahmebereich abzustrahlen und von dem Patient 100 emittierte Resonanzsignale zu empfangen und über eine Signalleitung ab-zugeben. Bevorzugter Weise wird aber die Körperspule 14 für das Aussenden des Hochfrequenzsignals und/oder das Empfangen durch Lokalspulen ersetzt, die in dem Patiententunnel 16 nahe am Patient 100 angeordnet sind. Es ist aber auch denkbar, dass die Lokalspule zum Senden und Empfangen ausgelegt ist und deshalb eine Körperspule 14 entfallen kann.

Eine Steuereinheit 20 versorgt die Magneteinheit 10 mit den verschiedenen Signalen für die Gradientenspulen 12 und die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus untereinander verbunden.

Das von Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar ist aber auch ein Aussenden des Hochfrequenzsignals über eine oder mehrere Lokalspulen.

Die Lokalspule empfängt dann vorzugsweise ein Magnetresonanzsignal aus dem Körper des Patienten 100, denn aufgrund des geringen Abstandes ist das Signal-zu-Rauschverhältnis (SNR) der Lokalspule besser als bei einem Empfang durch die Körperspule 14. Das von der Lokalspule empfangene MR-Signal wird in der Lokalspule aufbereitet und an die Hochfrequenzeinheit 22 des Magnetresonanztomographen 1 zur Auswertung und Bilderfassung weitergeleitet.

Die Bestrahlungsvorrichtung 200 ist in einer Relativposition zu dem Magnetresonanztomographen 9 so angeordnet, dass ein Strahl zu einem Zielgebiet in einem Aufnahmebereich des Magnetresonanztomographen 9 gelangen kann. Bei einem Split-Magnet ist es dabei auch denkbar, dass die Bestrahlungsvorrichtung 200 entlang einer Bahn um das Zielgebiet verfahren wird, um Schädigungen an einem Gewebe um das Ziel, z.B. einen Tumor, zu reduzieren.

Die Bestrahlungsvorrichtung 200 ist in der Lage, die abgegebene Strahlung in einer vorbestimmten Weise in der Intensität mit einer akustischen Frequenz modulieren, beispielsweise im Frequenzbereich von 1 Hz bis 100 kHz oder 10 Hz bis 10 kHz. Denkbar ist eine Röntgenquelle, oder ein Beschleuniger, bei dem die Intensität durch einen Strom der Ionen- bzw. Elektronenquelle veränderbar ist. Auch Strahlungsquellen mit einem Isotop wären denkbar, sofern durch einen steuerbaren Shutter eine Modulation bzw. Unterbrechung mit den genannten Frequenzen auf vorbestimmte Weise erzielbar ist.

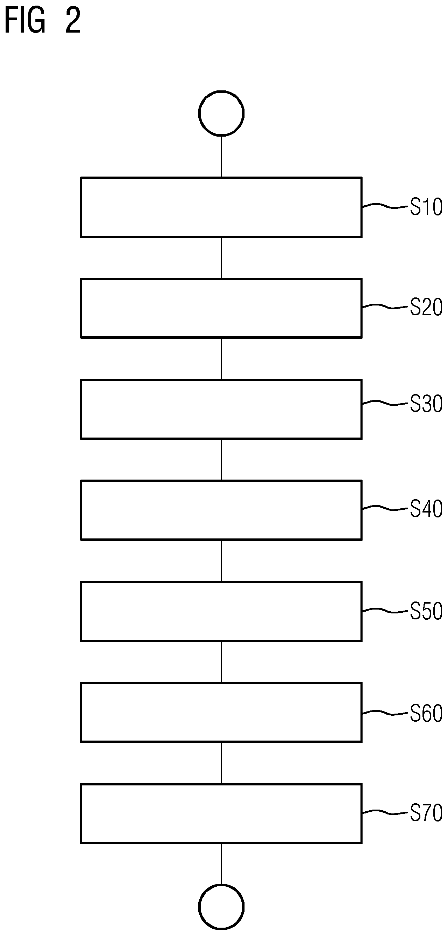

In Fig. 2 ist schematisch ein Ablaufdiagramm einer Ausführungsform des erfindungsgemäßen Verfahrens dargestellt.

In einem Schritt S20 wird eine intensitätsmodulierten Strahlung mittels der Bestrahlungsvorrichtung 200 in das Zielgebiet appliziert. Die Modulation erfolgt mit einer akustischen Frequenz, vorzugsweise in einem Bereich von 1 Hz bis 100 kHz oder 10 Hz bis 10 kHz. Dabei steht die Modulation in einem vorbestimmten Zeit- bzw. Phasenbezug zu einer Bildererfassung des Magnetresonanztomographen 9, mit anderen Worten, die Modulation ist mit der Bilderfassung synchronisiert. Die Synchronisation erfolgt mittels einer Signalverbindung 203 zwischen einer Steuerung 23 des Magnetresonanztomographen 9 und einer Steuereinheit 201 der Bestrahlungsvorrichtung 200.

In einem Schritt S30 erfasst der Magnetresonanztomograph 9 eine Magnetresonanzabbildung des Zielgebiets, während dem Einstrahlen. Unter "während" ist dabei zu verstehen, dass die Magnetresonanzabbildung zumindest den Zeitraum des Einstrahlens umfasst, sodass die Magnetresonanzabbildung eine gesamte eingestrahlte Strahlendosis erfassen kann. Vorzugsweise sind dabei Zeitabschnitte der Einstrahlung mit Zeitabschnitten der Bilderfassung synchronisiert bzw. erfolgen gleichzeitig, die sensitiv auf eine Auslenkung des Gewebes bzw. der darin enthaltenen Kernspins sind.

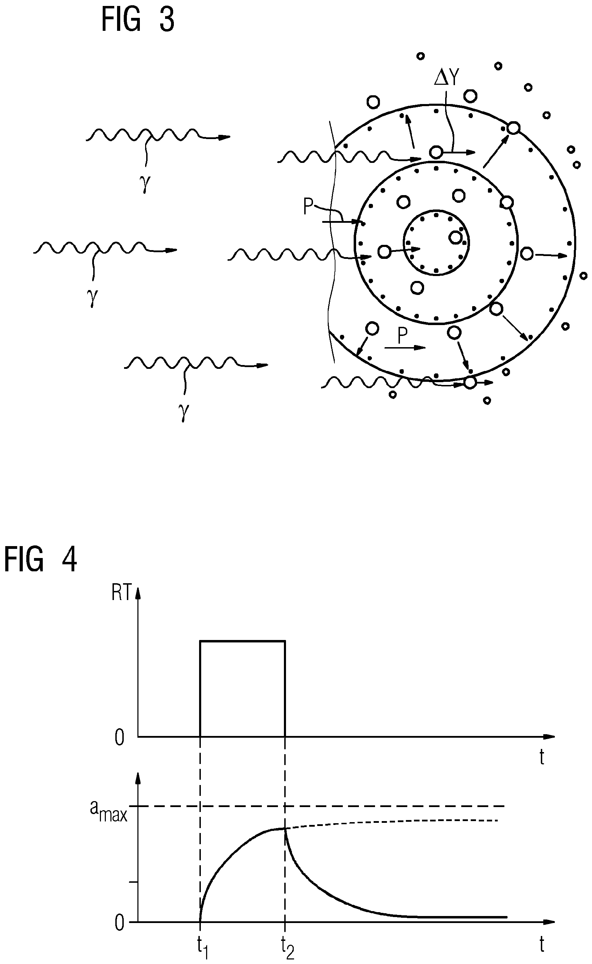

In Fig. 3 ist schematisch dargestellt, wie die einfallende Strahlung im Gewebe zu einer Auslenkung führt. Die einfallende Strahlung, seien es Röntgen- oder Gammaquanten oder auch hochenergetische Partikel, tragen zum einen Energie in das Gewebe ein, als auch einen Impuls P. Wird die Strahlung im Gewebe von den Atomen gestreut bzw. absorbiert, wird die Energie größtenteils in Wärme umgewandelt, die zu einer thermischen Ausdehnung führt, die Gewebe wie in Fig. 3 durch die konzentrischen Kreise angedeutet, um ein Zentrum mit einem maximalen Energieeintrag herum ausdehnen lässt. Durch die Ausdehnung erfolgt eine Bewegung von diesem Zentrum aus radial in alle Richtungen. Bei dem Ermitteln der Strahlendosis aus der erfassten Auslenkung sind diese geometrischen Zusammenhänge zu berücksichtigen, insbesondere wenn der thermische Effekt überwiegt.

Es findet aber auch ein Übertrag des Impulses P auf die Atome ganz oder teilweise statt und eine Kraft wird auf die Atome ausgeübt, die wiederum eine Bewegung entlang der Strahlrichtung bewirken kann, wie durch die Pfeile mit der Bezeichnung $\Delta y$ andeuten. Da die Atome elastisch in dem Gewebe gebunden sind, kann die Kraft ebenfalls zu einer Auslenkung führen. Welcher Effekt überwiegt, hängt unter anderem von der Art der Strahlung und deren Energie ab. Ionen-Strahlung beispielsweise führt aufgrund der hohen Ruhemasse der Partikel zu einem höheren Impulsübertrag.

Für die nicht schwingungsfähige Auslenkung lässt sich die Auslenkung wie folgt modellieren:

$$a(\vec{r}, t) = \begin{cases} a_{max} \cdot \left(1 - e^{-\frac{t-t_1}{\tau_{rise}}}\right) & , & t_1 \leq t \leq t_2 \\ a_{pk} \cdot e^{-\frac{t-t_{OFF}}{\tau_{decay}}} & , & t > t_2 \end{cases}$$

Die Zeitkonstanten $\tau_{rise}$ und $\tau_{decay}$ betreffen die elastischen Eigenschaften des Gewebes. In Muskelgewebe ist die Anstiegszeitkonstante $\tau_{rise}$ beispielsweise 3.2 ms und die Abfallzeitkonstante $\tau_{decay}$ 5.5 ms. In Lebergewebe ist die Anstiegszeit 6.2 ms und die Abfallzeit 7.9 ms. Der Parameter $a_{max}$ ist das quasistatische Maximum der Auslenkung für andauernde Bestrahlung.

Mit dem Parameter $a_{pk} = a_{max} \cdot \left(1 - e^{-\frac{t_2-t_1}{\tau_{rise}}}\right)$ ist die maximale Auslenkung des Gewebes angegeben, die am Ende eines Strahlenpulses erreicht wird.

In Fig. 4 ist schematisch der zeitliche Zusammenhang zwischen der einfallenden Strahlung und der davon hervorgerufenen Gewebeauslenkung dargestellt. Die modulierte Strahlung wird der Einfachheit halber als Rechteck-Impuls dargestellt, der durch Einschalten der Strahlungsquelle zu einem vorbestimmten Zeitpunkt t1 und Ausschalten zu einem vorbestimmten Zeitpunkt t2 definiert ist.

Der thermische Effekt wird in seinem zeitlichen Verlauf vor allem durch die Wärmekapazität des Gewebes und dessen Wärmeleiteigenschaften bestimmt. Die Temperatur, damit die Wärmeausdehnung und Auslenkung eines Volumenelements nimmt mit zunehmender Temperatur durch die von der Strahlung zugeführte Energie zu. Gleichzeitig nimmt der Wärmeabfluss mit der Temperaturdifferenz zu. Die Auslenkung nimmt zunächst steil zu und geht asymptotisch zu einem Gleichgewichtszustand über, sofern nicht vorher der Strahl unterbrochen wird. Nach der Unterbrechung nimmt die Temperatur durch Wärmediffusion wieder mit asymptotischem Verlauf zur Körpertemperatur ab.

Eine Kraftwirkung durch einen Impuls der Strahlung erfolgt hingegen instantan mit dem Einsetzen der Strahlung und ist näherungsweise konstant über die Zeit. Das Gewebe folgt dabei im Wesentlichen dem Modell eines gedämpften Pendels. Die Dämpfung wird verursacht durch die Viskosität des Gewebes, die Rückstellkraft durch die Elastizität des Gewebes für transversale Bewegung. In Fig. 4 ist der Fall dargestellt, dass die Viskosität des Gewebes zu einer asymptotischen Schwingung führt. Mit Einschalten der Strahlung geht die Auslenkung asymptotisch gegen einen maximalen Gleichgewichtswert $a_{max}$, und fällt nach Abschalten des Strahls asymptotisch wieder gegen 0 ab.

Insofern ist der beispielhafte Verlauf der Auslenkung in Fig. 4 für beide Fälle qualitativ vergleichbar und hängt im Wesentlichen von den Eigenschaften des Strahls, des Gewebes und der Strahldauer ab.

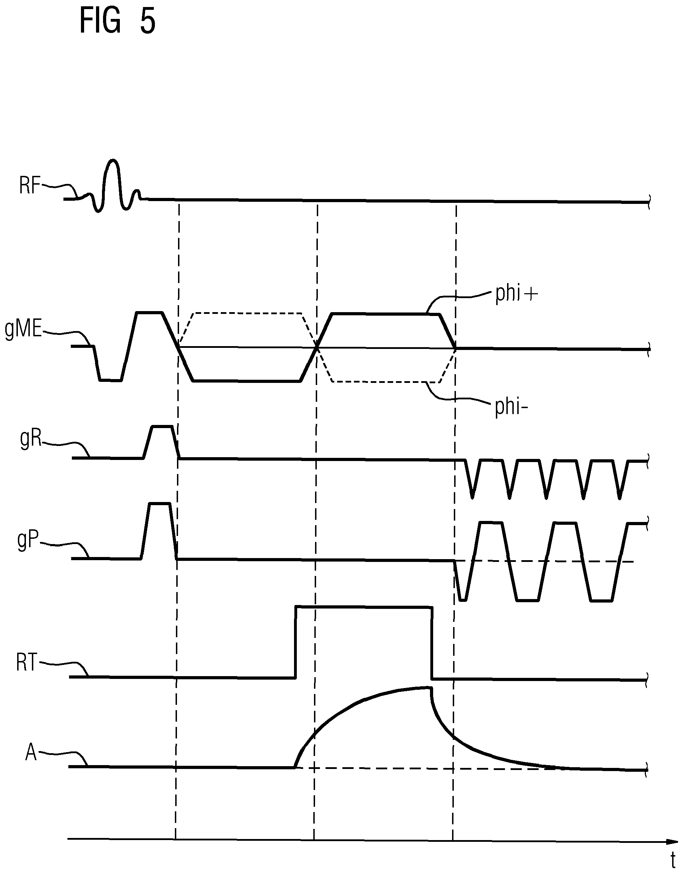

Unterschiedliche Beispiele für geeignete Sequenzen und Zeitabfolgen sind zu den nachfolgenden Figuren Fig. 5 bis Fig. 7 angegeben.

In einem Schritt S50 wird eine Dosisverteilung der Strahlung in dem Zielgebiet in Abhängigkeit von der erfassten Amplitude der Auslenkung ermittelt. Im einfachsten Fall ist die Auslenkung proportional zur Strahlungsintensität bzw. zur Leistung. Die Strahlendosis ist folglich proportional zu einem Integral der Auslenkung über die Zeit. Wie nachfolgend zu den Sequenzen erläutert, kann das Integral bereits durch die Sequenz realisiert sein. Alternativ ist es denkbar, das Integral numerisch auszuführen. Ein Proportional-Faktor kann beispielsweise durch eine Kalibrierungssequenz an einem Phantom mit einer definierten Strahlungsdosis erfolgen.

Vorzugsweise wird in einem Schritt S40 eine Referenz-Magnetresonanzabbildung des Zielgebiets mit dem Magnetresonanztomographen 9 ohne Einstrahlen durch die Bestrahlungsvorrichtung 200 erfasst. Auf diese Weise wird ein Referenzbild erfasst, dass durch den Patienten 100 und Inhomogenitäten der Bilderfassung des Magnetresonanztomographen 9 verursachte Einflüsse wiedergibt. Beispielsweise durch Differenzbildung in Schritt S50 Ermitteln der Dosisverteilung zwischen der Magnetresonanzabbildung mit Einstrahlen und Referenz-Magnetresonanzabbildung ohne Einstrahlen können diese Inhomogenitäten auf vorteilhafte Weise eliminiert werden.

Zum Erfassen der Auslenkung in der Magnetresonanzabbildung werden, wie nachfolgend noch näher zu den beispielhaften Sequenzen erläutert, sogenannte Motion-Encoding-Gradienten MEG verwendet, mit denen aus der Auslenkung des Gewebes eine Phasenabweichung der Magnetresonanzsignale hervorgerufen wird. Durch Suszeptibilitätssprünge im Gewebe können von den Motion-Encoding-Gradienten jedoch ähnliche Phasenveränderungen hervorgerufen werden. Da die Suszeptibilitätseffekte ihr Vorzeichen mit der Polarität der Gradienten im Gegensatz zu den durch die Auslenkung verursachten wechseln, werden in einer bevorzugten Ausführungsform die Schritte S30 des Erfassens der Magnetresonanzabbildung und S40 des Erfassens der Referenz-Magnetresonanzabbildung jeweils mit invertierter Polarität eines Phasenkodierungs-Gradienten wiederholt. Anschließend werden in Schritt S50 sowohl die Magnetresonanzabbildungen als auch die Referenz-Magnetresonanzabbildungen mit beiden Polaritäten der Motion-Encoding-Gradienten genutzt, um beim Bestimmen der Dosisverteilung die Suzeptibilitätseffekte zu kompensieren.

Es ist auch denkbar, dass in einem Schritt S10 mittels eines Hochfrequenzsignals des Magnetresonanztomographen eine vorbestimmte Erwärmung in dem Zielgebiet erfolgt. Beispielsweise kann durch eine Körperspule mit einer Vielzahl an Sendekanälen oder einem lokalen Sendearray eine vorbestimmte Feldverteilung erzielt werden, die durch Absorption der Hochfrequenzwellen eine vorbestimmte Erwärmung in dem Zielgebiet erzielt. Es ist aber auch denkbar, dass die vorbestimmte Erwärmung während oder nach Bestrahlen mit dem Strahlenpuls erfolgt.

Denkbar ist darüber hinaus, dass in einem Schritt S60 mittels einer Bilderfassung mit einer temperatursensitiven Sequenz eine Temperatur-Magnetresonanzabbildung des Zielgebiets zu erfasst und in einem weiteren Schritt S70 eine Temperaturkarte des Zielgebiets aus der Temperatur-Magnetresonanzabbildung ermittelt wird.

Nachfolgend sind einige mögliche Sequenzen dargestellt, die eine Sensitivität für das Erfassen einer Auslenkung aufweisen.

Fig. 5 stellt beispielhaft schematisch den zeitlichen Verlauf von Signalen einer GRE-EPI-Sequenz in zeitlicher Korrelation mit der Bestrahlung und deren erfassbarer Auswirkung auf das Gewebe.

Die Zeitachse verläuft von links nach rechts. Vertikale Linien sind eingefügt, um insbesondere gleichzeitige Ereignisse zu verdeutlichen. Die Sequenz ist nicht vollständig im Zeitablauf dargestellt, wie durch die Abbruchlinien rechts angedeutet ist, sondern nur ein repräsentativer Beginn der Sequenz.

Mit RF ist das Hochfrequenzsignal bezeichnet, das in den Patienten 100 zur Anregung der Kernspins eingestrahlt wird. In der GRE-EPI-Sequenz erfolgt zunächst ein Hochfrequenz-Puls zum Anregen einer vorbestimmten Schicht, gleichzeitig mit einem Schichtselektionsgardienten.

In den Fig. 5 bis 7 sind darunter drei Gradientensignale angetragen, gME, gR und gP. Es sind hier nicht x, y und z als Bezeichnungen gewählt, da die Achsen prinzipiell vertauschbar sind, je nachdem in welche Richtung eine Auslenkung erfasst werden soll.

gME bezeichnet hier den Gradienten, der zum Motion-Encoding verwendet wird. Für die GRE-Sequenz erfolgt das Motion-Encoding mit dem gleichen Gradienten, der auch zur Schicht-Selektion bei der Anregung der Kernspins genutzt wird. Mit gR wird der Gradient bezeichnet, der zum Readout genutzt wird, und mit gP der zur Phasencodierung genutzte Gradient. Die Gradienten der von den Gradientenspulen erzeugten Vektorenfelder spannen vorzugsweise ein kartesisches Koordinatensystem auf, wobei eine 2D-Schicht in der von gR und gP aufgespannten Ebene abgetastet wird. Die Stärke der Gradienten ist in willkürlichen Einheiten angetragen, wobei positive und negative Polaritäten bezüglich der Ruhelinie angegeben sind.

RT gibt den zeitlichen Verlauf der Strahlungsintensität der Bestrahlungsvorrichtung 200 in willkürlichen Einheiten an. Der Einfachheit halber wird hier ein Puls durch Ein- und Ausschalten des Strahls angenommen.

Mit A ist die Amplitude der Auslenkung im Gewebe angegeben. Wie zur Fig. 4 bereits erläutert, setzt die Auslenkung mit der Strahlung ein und fällt nach Ende der Strahlung wieder in die Ruheposition zurück.

In der GRE-EPI-Sequenz erfolgt die Codierung durch gME in einem Abschnitt ohne Strahlung bzw. dadurch induzierte Auslenkung mit einer Polarität des Gradienten und in einem Abschnitt mit Strahlung bzw. Auslenkung mit einer inversen Polarität des Gradienten. Durch Differenzbildung der Signale können dabei nicht-auslenkungsbedingte Effekte reduziert werden und so die Sensitivität für die Auslenkung erhöht werden. In Wiederholung der dargestellten Sequenz erfolgt eine Abtastung mit invertierten Polaritäten, wie durch die mit phi+ und phi- bezeichneten Kurvenverläufe angedeutet ist. Die Auslenkung kann so mit unterschiedlichen Gradientenfeldrichtungen erfasst werden. Derartige sogenannte Balanced Gradient-Pulse erlauben es, die Effekte der Auslenkung von Suszeptibilitätssprüngen induzierten Signalen zu unterscheiden, da die Richtung bzw. Polarität der von Suszeptibilitätssprüngen induzierten Signalen mit der umgekehrten Polarität ebenfalls die Richtung bzw. Polarität ändert. Auf diese Weise können auch Suszeptibilitätseffekte durch Differenzbildung reduziert werden.

Für eine Phasenänderung von Kernspins ergibt sich folgender Zusammenhang:

$$\Delta\varphi(\vec{r}, t) = \gamma \int_0^t \overrightarrow{MEG}(\vec{r}, \tau) \cdot \vec{u}(\vec{r}, \tau) \, d\tau$$

Mit $\varphi$ für die Phase, $\vec{r}$ für den Ort eines Kernspins, $\overrightarrow{MEG}(\vec{r},\tau)$ der Gradient gME in Abhängigkeit von Ort und Zeit, $\vec{u}(\vec{r},\tau)$ als Vektor für die Auslenkung eines Kernpins in Abhängigkeit von Ruheort und Zeit sowie $\gamma$ als gyromagnetischem Faktor.

Für bipolare und sog. "balanced" MEG und ohne Auslenkung im Gewebe während der MEG ist der Term gleich Null.

Die Sequenz wird vorzugsweise 4 mal wiederholt, ein erstes Mal mit positive MEG-Pulsen den Schichtselektionspulsen überlagert (in der Fig. 5 bei gME mit phi+ bezeichnet), ein zweites Mal mit positivem MEG, aber ohne Strahlungs-Pulse RT, das dritte Mal mit negativem MEG-Pulsen (in der Fig. 5 bei gME mit phi- bezeichnet) und das vierte Mal wieder ohne Strahlungspulse RT. Die vier kumulativen Phasenverschiebungs-Karten, die respektive mit diesen vier Magnetresonanzsequenzen erfasst werden sind:

$$\begin{cases} \emptyset^+(x,y) & \textit{mit positivem MEG} \\ \emptyset^{0+}(x,y) & \textit{und ohne RT pulse} \\ \emptyset^-(x,y) & \textit{mit negativem MEG} \\ \emptyset^{0-}(x,y) & \textit{und ohne RT Pulse} \end{cases}$$

Die Karten mit dem Phasenhintergrund, erfasst ohne Strahlungspuls, liefern die Kalibrierungsdaten zur Kompensation für die Effekte von Wirbelströmen, da die EPI-Sequenz sehr empfindlich auf Streufelder von Leitern in der Umgebung sind, die von den Gradientenfeldern hervorgerufen werden.

So ergibt sich für die Auslenkungskarte des Gewebes:

$$a(x,y) = \frac{\emptyset^+(x,y) - \emptyset^{0+}(x,y) - \emptyset^-(x,y) + \emptyset^{0-}(x,y)}{\gamma \cdot G_{MEG} \cdot 2\delta}$$

Wobei $G_{MEG}$ die Amplitude und $2\delta$ ist die Dauer der MEG-Pulse Daraus ergibt sich eine Temperaturkarte mit

$$\Delta T(x,y) = \frac{\emptyset^+(x,y) - \emptyset^{0+}(x,y) + \emptyset^-(x,y) - \emptyset^{0-}(x,y)}{\gamma \cdot TE \cdot B_0 \cdot 2\alpha}$$

wobei $\alpha$ = -0.0094 ppm/°C der Temperaturkoeffizient für die Verschiebung der Protonenresonanzfrequenz (PRFS), TE die Echozeit und B0 die Stärke des statischen Magnetfeldes B0 in Tesla ist.

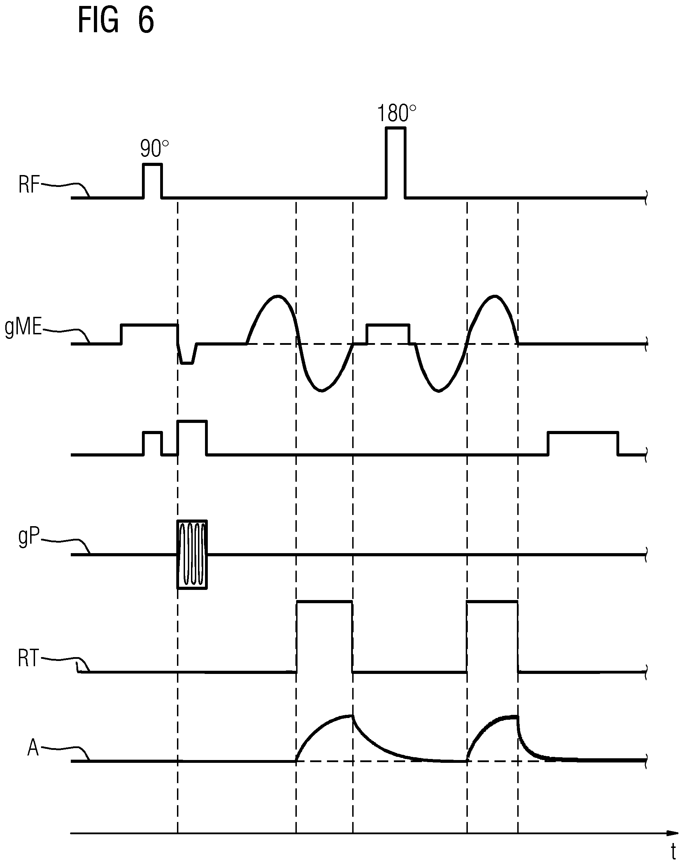

Fig. 6 zeigt eine beispielhafte, für die Sensitivität auf Auslenkung des Gewebes modifizierte standard-Magnetresonanzsequenz SE.

Die RF-Hochfrequenzpulse weisen einen 90° Puls auf, um die Magnetisierung in die transversale Ebene zu kippen, gefolgt von einem 180° Refokussierungspuls, beide mit der Larmorfrequenz bzw. Resonanzfrequenz ausgeführt (z.B. 64MHz bei einem 1.5T Magnetresonanztomographen).

Zusätzliche sind die Auslenkungssensitivität steigernde Gradienten in der Standard-Sequenz in Schichtselektionsrichtung ergänzt, um die strahlungsbedingte Auslenkung zu enkodieren. Diese sind auf gME als sinusförmige Pulse dargestellt.

Ein zweiter Strahlungspuls wird synchron zur zweiten MSG Periode ausgelöst, um eine synchrone Auslenkung des Gewebes zu verursachen und die Strahlungspulsdauer zu minimieren. Die Intensität und die Dauer des Strahlungspulses RT wird so gewählt, dass eine ausreichende Auslenkung des Gewebes für die Erfassung durch die Sequenz hervorgerufen wird, gleichzeitig aber gering genug, um Gewebeschäden zu minimieren. Die MR-Signale mit der akkumulierten Phasenverschiebung werden dann während des Readout-Fensters erfasst.

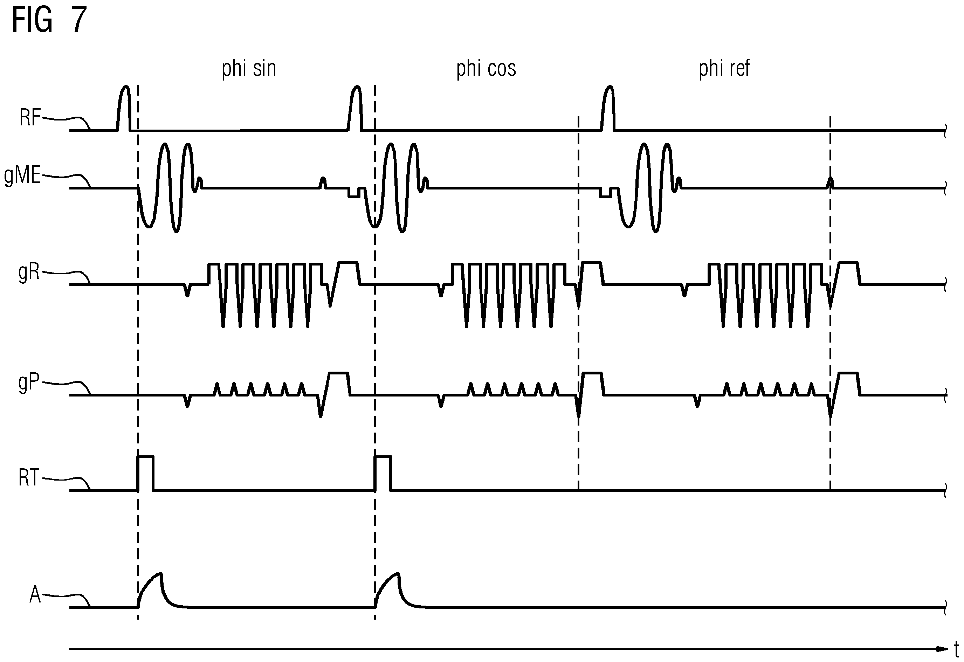

In Fig. 7 ist eine weitere modifizierte EPI-Sequenz dargestellt, die den Einfluss weiterer nicht strahlungsinduzierter Auslenkungen des Gewebes berücksichtigt, beispielsweise durch Atmung oder Herzschlag. Die schnelle Abfolge von drei EPI-Sequenzen erfasst die Phasenakkumulation in dem rekonstruierten Magnetresonanzabbildungen mit Sinus- und Cosinus-modulierten MEG-Pulsen und einer dritten Magnetresonanzabbildung mit MEG-Pulsen, aber ohne Strahlungspulse. Diese letzte Sequenz misst den akkumulierten Phasenhintergrund, verursacht durch andere Arten von langsamerer Bewegung. Auf vorteilhafte Weise werden durch die Sinus- bzw. Cosinus-modulierten Gradientenpulse sowohl Wirbelstromeffekte reduziert als auch der durch die Gradientenpulse abgestrahlte akustische Lärm.

Im Rahmen der Erfindung sind auch weitere Variationen von Sequenzen denkbar, die eine Sensitivität auf Gewebeauslenkungen bewirkt. Insbesondere sind andere, im Rahmen der Elastografie bereits genutzte Sequenzen denkbar. Ebenfalls sind Sequenzen denkbar, die im Rahmen von Bilderfassung mit Kontrastmitteln angewendet werden.

Weiterhin sind Sequenzen denkbar, die zur Diffusionsmessung genutzt werden, da diese insbesondere nicht auf eine vorbestimmte lineare Bewegungsrichtung limitiert sind, sondern auch eine kugelsymmetrische Bewegung durch die thermische Expansion erfassen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren zur Echtzeitüberwachung einer von einer Bestrahlungsvorrichtung applizierten Strahlendosis in einem Zielgebiet mittels eines Magnetresonanztomographen, wobei die Bestrahlungsvorrichtung ausgebildet ist, die Strahlung in einer in der Intensität im akustischen Frequenzbereich modulierten Form abzugeben, wobei der Magnetresonanztomograph ausgebildet ist, eine Magnetresonanzabbildung des Zielgebiets während der Bestrahlung zu erfassen, wobei das Verfahren die Schritte aufweist:

   (S20) Einstrahlen einer modulierten Strahlung mittels der Bestrahlungsvorrichtung in das Zielgebiet;
   (S30) Erfassen einer Magnetresonanzabbildung des Zielgebiets während dem Einstrahlen mit dem Magnetresonanztomographen,
   wobei der Magnetresonanztomograph eine Sequenz anwendet, die ausgebildet ist, eine Amplitude einer Auslenkung eines Gewebes in dem Zielgebiet zu erfassen;
   (S50) Ermitteln einer Dosisverteilung der Strahlung in dem Zielgebiet in Abhängigkeit von der erfassten Amplitude der Auslenkung.

2. Verfahren nach Anspruch 1, wobei das Verfahren weiterhin den Schritt (S40) aufweist, eine Referenz-Magnetresonanzabbildung des Zielgebiets ohne Einstrahlen zu erfassen und in dem Schritt Ermitteln der Dosisverteilung die Dosisverteilung in Abhängigkeit von der Magnetresonanzabbildung und der Referenz-Magnetresonanzabbildung ermittelt wird.

3. Verfahren nach Anspruch 1 oder 2, wobei bei dem Schritt (S30) des Erfassens der Magnetresonanzabbildung und dem Schritt (S40) des Erfassens der Referenz-Magnetresonanzabbildung eine Sequenz mit Motion-Encoding-Gradienten verwendet wird und der Schritt des Erfassen einer Magnetresonanzabbildung und/oder der Schritt des Erfassens einer Referenz-Magnetresonanzabbildung erfolgt mit invertierter Polarität eines Phasenkodierungs-Gradienten.

4. Verfahren nach einem der vorhergehenden Ansprüche, wobei das Verfahren weiterhin den Schritt (S60) aufweist, mittels einer Bilderfassung mit einer temperatursensitiven Sequenz eine Temperatur-Magnetresonanzabbildung des Zielgebiets zu erfassen und in einem weiteren Schritt (S70) eine Temperaturkarte des Zielgebiets aus der Temperatur-Magnetresonanzabbildung ermittelt wird.

5. Verfahren nach einem der vorhergehenden Ansprüche, wobei aus einer der erfassten Magnetresonanzabbildungen eine Position eines Zielgewebes ermittelt wird und eine weitere Bestrahlung in Abhängigkeit von der ermittelten Position erfolgt.

6. Verfahren nach einem der vorhergehenden Ansprüche, wobei das Verfahren weiterhin den Schritt (S10) aufweist, mittels eines Hochfrequenzsignals des Magnetresonanztomographen eine vorbestimmte Erwärmung in dem Zielgebiet zu erzielen.

7. System zur Echtzeitüberwachung einer von einer Bestrahlungsvorrichtung applizierten Strahlendosis in einem Zielgebiet, wobei das System die Bestrahlungsvorrichtung und einen Magnetresonanztomographen aufweist,

   wobei die Bestrahlungsvorrichtung ausgebildet ist, die Strahlung in einer in der Intensität im akustischen Frequenzbereich modulierten Form synchronisiert mit einer Bilderfassung des Magnetresonanztomographen abzugeben und in das Zielgebiet einzustrahlen;
   wobei der Magnetresonanztomograph ausgebildet ist, eine Magnetresonanzabbildung des Zielgebiets während dem Einstrahlen zu erfassen, wobei der Magnetresonanztomograph eine Sequenz anwendet, die ausgebildet ist, eine Amplitude einer Auslenkung eines Gewebes in dem Zielgebiet zu erfassen;
   wobei das System ausgebildet ist, eine Dosisverteilung der Strahlung in dem Zielgebiet in Abhängigkeit von der erfassten Amplitude der Auslenkung des Gewebes zu ermitteln.

8. Computerprogrammprodukt, welches ein Programm umfasst und direkt in einen Speicher einer programmierbaren Steuerung (23) eines Systems nach Anspruch 7 ladbar ist, mit Programmmitteln, um alle Schritte des Verfahrens nach einem der Ansprüche 1 bis 6 auszuführen, wenn das Programm in der Steuerung (23) ausgeführt wird.

9. Elektronisch lesbarer Datenträger mit darauf gespeicherten elektronisch lesbaren Steuerinformationen, welche derart ausgestaltet sind, dass sie bei Verwendung des Datenträgers in einer Steuerung (23) eines Systems nach Anspruch 7 das Verfahren nach einem der Ansprüche 1 bis 6 durchführen.

# FIG 1

# FIG 2

## FIG 3

## FIG 4

# FIG 5

RF

gME                                     phi+

                                         phi-

gR

gP

RT

A

                                         t

# FIG 6

# FIG 7

EP 4 574 205 A1

Europäisches
Patentamt

European
Patent Office

Office européen
des brevets

# EUROPÄISCHER RECHERCHENBERICHT

**Nummer der Anmeldung**

EP 23 21 9623

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2017/165504 A1 (DOLLINGER GÜNTHER [DE] ET AL) 15. Juni 2017 (2017-06-15) * Absätze [0015], [0017], [0029]; Anspruch 1; Abbildung 2 * ----- | 1-9 | INV. A61N5/10 G01R33/48 |
| A | PLEWES D B ET AL: "QUANTITATIVE MAGNETIC RESONANCE IMAGING OF ULTRASOUND FIELDS", PROCEEDINGS OF THE 1997 IEEE ULTRASONICS SYMPOSIUM. ONTARIO, CANADA, OCT. 5 - 8, 1997; [IEEE ULTRASONICS SYMPOSIUM PROCEEDINGS], NEW YORK, NY : IEEE, US, 5. Oktober 1997 (1997-10-05), Seiten 1297-1300, XP000800013, ISBN: 978-0-7803-4154-8 * Zusammenfassung; Abbildungen 1-4 * ----- | 1-9 | |
| A | US 2023/277875 A1 (SCHAUER JANNIS [DE] ET AL) 7. September 2023 (2023-09-07) * Absatz [0129]; Anspruch 1; Abbildung 2 * ----- | 1-9 | |

**RECHERCHIERTE SACHGEBIETE (IPC)**

A61N
G01R

Der vorliegende Recherchenbericht wurde für alle Patentansprüche erstellt

| Recherchenort | Abschlußdatum der Recherche | Prüfer |
|---|---|---|
| München | 24. Mai 2024 | Kajzar, Anna |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 21 9623

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-05-2024

| Im Recherchenbericht angeführtes Patentdokument | Datum der Veröffentlichung | Mitglied(er) der Patentfamilie | Datum der Veröffentlichung |
|---|---|---|---|
| US 2017165504 A1 | 15-06-2017 | EP 2974771 A1<br>US 2017165504 A1<br>WO 2016009042 A1 | 20-01-2016<br>15-06-2017<br>21-01-2016 |
| US 2023277875 A1 | 07-09-2023 | EP 4238610 A1<br>US 2023277875 A1 | 06-09-2023<br>07-09-2023 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012211581 A1

**In der Beschreibung aufgeführte Nicht-Patentliteratur**

- Real-time, volumetric imaging of radiation dose delivery deep into the liver during cancer treatment. *Nature Biotechnology*, https://www.nature.com/articles/s41587-022-01593-8